# EUROPEAN PATENT APPLICATION

(11) **EP 3 023 827 A1**
(43) Date of publication of application: **25.05.2016**
(21) Application number: 14194034.6
(22) Date of filing: 20.11.2014
(51) Int. Cl.: G02B 27/01, G02B 27/00, A61B 3/113, H04N 13/04, G06F 3/01, G06K 9/00, G03B 21/00

(54) **Head mountable device for measuring eye movement having visible projection means**

(71) Applicant: GN Otometrics A/S, 2630 Taastrup (DK)
(72) Inventor: Macdougall, Hamish, Woolloomooloo, New South Wales 2011 (AU)
(74) Representative: Zacco Denmark A/S

(57) **Abstract**

Disclosed is a method and a head mountable device for measuring eye movement of a user, the head mountable device comprising: a frame; a camera system comprising a first camera, wherein the camera system is configured to obtain a first set of images of a first eye of the user; a projection system for projecting a visible object in a field of view of the first eye when the user wears the head mountable device. The projection system is configured to perform a movement of the visible object relative to the head mountable device. The projection system comprises a first projector configured to project a first projection comprising the visible object.

## Description

### FIELD

The present disclosure relates to a device for measuring eye movement, in particular a head mountable device for measuring eye movement in relation to tests involving visible tracking of an object. Such tests may be ophthalmologic, neurologic, and/or perception tests.

### BACKGROUND

There is an ongoing investigation towards developing measurement techniques and equipment for measuring eye movement. Various ophthalmologic, vestibular, perception, and neurologic tests exists which involves observing eye movements. Tests may comprise observation of eye movements of a user while the user focus on an object, such as an object that moves within an area visible to the user. Conventionally a clinician may ask the user to focus on the clinician's index finger while the clinician is moving his index finger around in front of the user. Tests incorporating such visible tracking of an object may comprise ophthalmologic, neurologic, and/or perception tests.

Tests may comprise measuring fast eye movements, e.g. eye saccades, lasting approximately between 20-200 ms and involving angular speed up to 900 deg/s. Such fast movements may be visible to the clinician, but may be difficult to quantify consistently.

It is desirable to circumvent subjective measurements and provide a method, such as a possible standardized test, which is independent of the clinician or other person performing the test. Furthermore, in some environments, such as in pre hospital settings, it may be problematic, if not impossible, to accurately perform a test when relying on subjective measurements.

Furthermore, it is desirable that a device capable of performing the above mentioned method or tests is able to control the object to be visibly tracked in a consistent, secure, comfortable, and easy way.

### SUMMARY

There is a need for an improved device which avoids the use of subjective measures in ophthalmologic, vestibular, perception and/or neurologic tests, and which avoids or limits the need of user interaction during tests, and hence is able to reliably measure eye movement when performing various tests. The present disclosure provides a device and a method which provides objective and reproducible measurement of eye movement in tests requiring visibly tracking of an object.

Disclosed is a head mountable device for measuring eye movement of a user. The head mountable device comprising: a frame; a camera system; and a projection system. The camera system comprises a first camera, and the camera system is configured to obtain a first set of images of a first eye of the user. The projection system is configured for projecting a visible object in a field of view of the first eye, and/or in a field of view of a second eye, when the user wears the head mountable device. The projection system is configured to perform a movement of the visible object relative to the head mountable device. The projection system comprises a first projector configured to project a first projection comprising the visible object.

Also disclosed is a method for measuring eye movement of a user wearing a head mountable device comprising a frame; a camera system comprising a first camera, and a projection system comprising a first projector. The method comprising: obtaining a first set of images of a first eye of the user by the camera system; projecting by the projection system a first projection comprising a visible object in a field of view of the first eye and/or in a field of view of a second eye; and performing a movement of the visible object relative to the head mountable device by the projection system.

The head mountable device used in the method may be the head mountable device for measuring eye movement as also disclosed. The method may be implemented with the device for measuring eye movement. At least a part of the method may be incorporated in software adapted to run in a processing unit, such as a processing unit of the device for measuring eye movement.

It is envisaged that any embodiments or elements as described in connection with any one aspect may be used with any other aspects or embodiments, mutatis mutandis.

The method and apparatus as disclosed provide a head mountable device for measuring eye movement, featuring elements which allow automated procedures for performing ophthalmologic, vestibular, and/or neurologic tests involving visible tracking of an object. Thus, the method and apparatus disclosed enable fast and objective examination of ophthalmologic, vestibular and neurologic parameters. Objective examinations as an alternative to conventional subjective assessments may provide more reliable and consistent examinations. Hence, incorrect or unnecessary treatment may be avoided, and improved possibility of detecting changes in a patient's condition is provided.

The head mountable device comprises a frame. The frame may be configured to be fixated to the head of the user, e.g. by adjustable and/or elastic straps. The frame may be in the form a goggle, a helmet, a cap, and/or another head mountable equipment. In an embodiment, the frame is embodied as a goggle. The frame may be configured to fasten the head mountable device to the head of the user such as to prevent motion of the head mountable device relative to the head of the user. The frame may accommodate elements of the head mountable device. The frame may accommodate the camera system and/or the projection system.

The method may further comprise mounting the head mountable device and/or the frame to the head of the user.

The head mountable device may be operable without attached wires. The head mountable device may comprise a power supply, such as a battery power supply and/or a power inlet. The frame may accommodate power supply. The power supply may be attached to the frame. Providing a power supply may allow operation of the head mountable device without the need of a power outlet, thus providing an increased scope of operation, e.g. the head mountable device may be used in an ambulance or at an accident site.

The method may comprise projecting the first projection on a surface in front of the user, e.g. a wall, a ceiling, and/or a screen. The projection system may be configured to project the first projection on the surface in front of the user.

The movement of the visible object performed by the projection system may be achieved in various ways. The projection system may comprise a first motor configured to perform the movement of the visible object by changing a first projection direction of the first projector. The projection system may comprise a second motor configured to perform the movement of the visible object by changing the first projection direction of the first projector. The first motor may change the first projection direction along a first direction. The second motor may change the first projection direction along a second direction. The first direction and the second direction may be non-parallel. For example, the first direction and the second direction may be perpendicular.

The first motor and/or the second motor may be a stepper motor. The first motor and/or the second motor may be a servo motor.

The first motor and/or the second motor may change the first projection direction by changing an orientation, such as the pointing direction, of the first projector. Alternatively or additionally, the first motor and/or the second motor may change the first projection direction by changing an orientation of an element, such as a mirror or a lens interacting with the first projection.

The projection system may comprise a projection mirror. The projection mirror may be configured to interact with the first projection, such as to reflect the first projection. The projection mirror may direct the first projection in a desired direction, such as in a direction towards the field of view of the first eye and/or the field of view of the second eye. The projection mirror may provide increased design freedom for positioning of the first projector, e.g. positioning of the first projector and/or the projection system on the frame.

The first motor and/or the second motor may change the first projection direction by changing an orientation of the projection mirror. For example, the first motor may tilt the projection mirror about a first mirror axis and/or the second motor may tilt the projection mirror about a second mirror axis. The first mirror axis and the second mirror axis may be non-parallel. For example, the first mirror axis and the second mirror axis may be perpendicular.

The projection system may comprise an optical lens. The optical lens may be configured for changing a shape of the visible object. The optical lens may be configured to interact with the first projection, such as to change the shape of the visible object. The optical lens may be an electrically modifiable lens. For example, the optical lens may comprise liquid crystals, and the optical lens may be configured to change lens properties, e.g. a refractive index, such as a refractive index of the liquid crystals, by subjecting the liquid crystals to an electrical field.

The projection system may comprise a motor, such as the first motor, the second motor, and/or a third motor, configured to change the position of the optical lens, e.g. changing the position of the optical lens relative to the first projector. The projection system may comprise a plurality of optical lenses including the optical lens.

The first projection may be an image, e.g. a pattern and/or a photograph and/or a drawing and/or a computer graphic. For example, the first projection may be an image comprising the visible object. For example, the visible object may be a balloon, a car, a ball, and/or an animal etc.

The first projector may be configured to perform the movement of the visible object by providing a sequence of projections including the first projection. For example, the first projector may be a video projector and/or the first projection may be an image of a sequence of images, such as a video.

The sequence of projections may comprise a plurality of projections including the first projection. The plurality of projections may comprise a second projection, a third projection, a fourth projection, and/or a fifth projection. The first projection, the second projection, the third projection, the fourth projection, and/or the fifth projection may be frames of the sequence of images. The sequence of images may be a film with a frame rate, such as a frame rate of 12 frames per second, or 24 frames per second, or 25 frames per second, or 30 frames per second, or 48 frames per second, or more than 24 frames per second, such as more than 100 frames per second.

The first projection and/or the plurality of projections may be an image with a resolution. The resolution may be more than 64x64 pixels, such as, or more than, 640x480 pixels, such as, or more than, 768x576 pixels, such as, or more than, 800x600 pixels, such as, or more than 1280x720 pixels, such as, or more than 1600x1200 pixels, such as, or more than, 1920x1080 pixels.

The first projection and/or the visible object may be a dot. For example, the first projector may be a laser pointer.

The first projection and/or the visible object may be a dot, e.g. a dot having a diameter. The diameter may be more than 0.1 cm, such as more than 0.5 cm, such as more than 1 cm. The diameter may be less than 10 cm, such as less than 5 cm, such as less than 2 cm. The diameter may be dependent on a distance to a surface e.g. a wall, a ceiling, and/or a screen, whereon the first projection and/or the visible object is projected. The diameter may be more than 0.1 cm, such as more than 0.5 cm, such as more than 1 cm and/or the diameter may be less than 10 cm, such as less than 5 cm, such as less than 2 cm, when the distance to the surface whereon the first projection and/or the visible object is projected is between 0.5 and 5 meters.

The projection system may be configured for projecting a visible object in a field of view of the second eye when the user wears the head mountable device. The visible object projected in the field of view of the second eye, may be the visible object projected in the field of view of the first eye. Alternatively and/or additionally, the projection system may be configured for projecting a first visible object in the field of view of the first eye, and a second visible object in the field of view of the second eye.

In some tests, the visible object may be projected in the field of view of the second eye and the camera system may be configured to obtain the first set of images of the first eye. Such a setup may allow measuring eye movement of the eye opposite the eye tracking the visible object. In other tests the visible object may be projected in the field of view of the first eye and the camera system may be configured to obtain the first set of images of the first eye. Such a setup may allow measuring eye movement of the eye tracking the visible object. The head mountable device as disclosed may comprise both of the above setups providing an option of measuring the eye tracking the visible object and/or measuring the eye opposite to the eye tracking the visible object.

In some tests, it may be beneficial to be able to obtain images of both eyes of a user. Hence, the camera system may be configured to obtain a second set of images of a second eye of the user. The first camera may be configured to obtain the first set of images and the second set of images. Alternatively and/or additionally, the camera system may comprise a second camera configured to obtain the second set of images.

The first set of images may be configured to be obtained with a first frame rate. The first frame rate may be selected such as to enable detection of eye saccades of the first eye. The second set of images may be configured to be obtained with a second frame rate. The second frame rate may be selected such as to enable detection of eye saccades of the second eye. The first frame rate and the second frame rate may be the same frame rate or may be different frame rates.

Obtaining the first set of images and/or the second set of images preferably enable detection of eye saccades of the first eye and/or of the second eye. Eye saccades may be very fast, e.g. eye saccades may last for only 20 ms. Therefore, the first frame rate and/or the second frame rate may be sufficiently high to enable reliable detection of eye saccades. For example, the first frame rate and/or the second frame rate may be higher than 125 frames per second (fps), such as higher than 150 fps, such as higher than 175 fps, such as higher than 200 fps, such as 250 fps. In other examples, the first frame rate and/or the second frame rate may be less than 125 fps, but is still sufficiently high to allow the processing unit to detect eye saccades of the first eye and/or of the second eye.

The head mountable device may comprise a first mirror for mirroring images of the first eye towards the first camera, and/or for mirroring images of the first eye towards the second camera, and/or for mirroring images of the second eye towards the first camera, and/or for mirroring images of the second eye towards the second camera. Additionally, the head mountable device may comprise a second mirror for mirroring images of the second eye towards the first camera and/or for mirroring images of the second eye towards the second camera.

The frame may accommodate the first mirror and/or the second mirror.

The first camera and/or the second camera may be focused on the first and/or second eye. The first camera and/or the second camera may be focused on the first and/or second eye via the first and/or second mirror.

The head mountable device may comprise a first light source for emitting first electromagnetic radiation towards the first eye and/or the second eye. The first mirror and/or the second mirror may be configured to direct at least a part of the first electromagnetic radiation towards the first eye and/or the second eye.

The head mountable device may comprise a second light source for emitting second electromagnetic radiation towards the first and/or second eye. The first mirror and/or the second mirror may be configured to direct at least a part of the second electromagnetic radiation towards the first eye and/or the second eye.

The frame may accommodate the first light source and/or the second light source.

The first and/or second electromagnetic radiation may comprise infrared radiation, laser radiation, visible red radiation, visible blue radiation, visible green radiation, and/or visible orange radiation. The first and/or second electromagnetic radiation may comprise electromagnetic radiation with wavelengths in the range of 380-450 nm, or in the range of 450-495 nm, or in the range of 495-570 nm, or in the range of 570-590 nm, or in the range of 590-620 nm, or in the range of 620-750 nm, or in the range of 750-2.500 nm, or in the range of 2.500-10.000 nm, or in the range of 10.000-1.000.000 nm.

The first and/or second light source may be used for testing the first and/or second eye's response to light. The first and/or second light source may be used to light up the first and/or second eye. The first and/or second light source may be used to light up the first and/or second eye for the camera system to obtain images of the first and/or second eye. The camera system and/or the first camera and/or the second camera may be configured to detect the first electromagnetic radiation and/or the second electromagnetic radiation.

The first and/or second mirror may be partly transparent. For example, the first and/or second mirror may be transparent to one or more selected ranges of electromagnetic radiation. The first and/or second mirror may be transparent to visible light, such as electromagnetic radiation with wavelengths in the range of 380-750 nm.

The head mountable device may comprise one or more processing unit(s), such as a first processing unit and/or a second processing unit.

The first processing unit may be configured to process the first set of images. The first processing unit may be configured to provide a processing unit output based on the first set of images.

The first processing unit and/or the second processing unit may be configured to control the projection system. The first processing unit and/or the second processing unit may be configured to control the projection system to perform the movement of the visible object relative to the head mountable device. For example, the first processing unit and/or the second processing unit may be configured to control the first motor and/or the second motor, and/or the first processing unit and/or the second processing unit may be configured to control the first projector.

The head mountable device may comprise a processing unit, such as the first processing unit, configured to process the first set of images and control the projection system.

The head mountable device may comprise an interface for providing a device output. The device output may be based on the first set of images and/or the second set of images. The method may comprise providing a device output based on the first set of images and/or the second set of images. The interface may comprise one or more types of interfaces for providing the device output to a user and/or an operator of the head mountable device.

The frame may accommodate the interface.

The interface may comprise one or more display(s), such as a first display and/or a second display. The one or more display(s), such as the first display and/or the second display, may be an organic light emitting diode (OLED), an OLED display, a light emitting diode (LED), an LED display, and/or an e-ink display. The one or more display(s), such as the first display and/or the second display, may visually provide the device output, or part of the device output, to a user or an operator. The device output may comprise a visual output.

The interface may comprise one or more speaker(s), such as a first speaker and/or a second speaker. The one or more speaker(s), such as the first speaker and/or the second speaker, may audiologically provide the device output, or part of the device output, to a user or an operator. The device output may comprise an audiologic output, such as sound.

The interface may comprise one or more wireless transmitter unit(s). The interface may comprise a wireless transceiver unit comprising the wireless transmitter unit and a wireless receiver unit. The wireless transmitter unit and/or the wireless transceiver unit and/or the wireless receiver unit may operate according to a wireless protocol, e.g. Bluetooth, WiFi, 3G, and/or 4G.

Providing the device output may comprise transmitting the device output wirelessly to an external display. The wireless transmitter unit may be configured to transmit the device output, or a part of the device output, to a display, such as an external display. The external display may be external to the head mountable device. The external display may be external to the frame of the head mountable device. The external display may be a display of a smartphone, a tablet computer, a laptop, a TV, a smart-TV, and/or the like.

The interface may comprise an input device for enabling control of the head mountable device, such as enabling control of the projection system. The input device may enable control of the projection system via control of the processing unit, such as the first processing unit and/or the second processing unit. The input device may be the wireless receiver. Alternatively or additionally, the input device may comprise a touch display, a push button and/or a switch.

The head mountable device may comprise additional measurement sensors, such as a first motion sensor configured to detect movement of the head mountable device. The frame may accommodate the additional measurement units, such as the motion sensor. The motion sensor may comprise one or more gyroscope(s) and/or one or more accelerometer(s) and/or one or more camera(s). Additional measurement units may provide additional uses of the head mountable device, e.g. the head mountable device may be configurable to be used in more tests.

The frame may accommodate any or all of the above mentioned elements. Hence, the head mountable device may be configured as a standalone device without the need for external connections.

The projection system may comprise a plurality of projectors including the first projector and a second projector. One or more of the features as described in relation to the first projector may apply to one or more of the plurality of projectors, such as to the first projector and/or to the second projector. For example, the second projector may be a video projector, and/or the second projector may be a laser pointer.

The plurality of projectors may be differently polarized. For example, the first projector may have a first polarization and the second projector may have a second polarization. The first polarization may be different than the second polarization, e.g. the first polarization may be 90 degrees rotated relative to the second polarization. The first projector may comprise a first polarizer having the first polarization and the second projector may comprise a second polarizer having the second polarization. The first polarization and/or the second polarization may be a linear polarization.

### BRIEF DESCRIPTION OF THE DRAWINGS

The above and other features and advantages will become readily apparent to those skilled in the art by the following detailed description of exemplary embodiments thereof with reference to the attached drawings, in which:
- Fig. 1: schematically illustrates an exemplary head mountable device,
- Fig. 2: schematically illustrates an exemplary head mountable device,
- Fig. 3: schematically illustrates an exemplary projection system,
- Fig. 4: schematically illustrates an exemplary projection system,
- Fig. 5: schematically illustrates an exemplary sequence of projections,
- Fig. 6: schematically illustrates an exemplary camera system,
- Fig. 7: schematically illustrates an exemplary camera system,
- Fig. 8: schematically illustrates an exemplary head mountable device,
- Fig. 9: schematically illustrates an exemplary interface,
- Fig. 10: is a flow diagram of a method for measuring eye movement.

### DETAILED DESCRIPTION

Various embodiments are described hereinafter with reference to the figures. Like reference numerals refer to like elements throughout. Like elements will, thus, not be described in detail with respect to the description of each figure. It should also be noted that the figures are only intended to facilitate the description of the embodiments. They are not intended as an exhaustive description of the claimed invention or as a limitation on the scope of the claimed invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

Throughout, the same reference numerals are used for identical or corresponding parts.

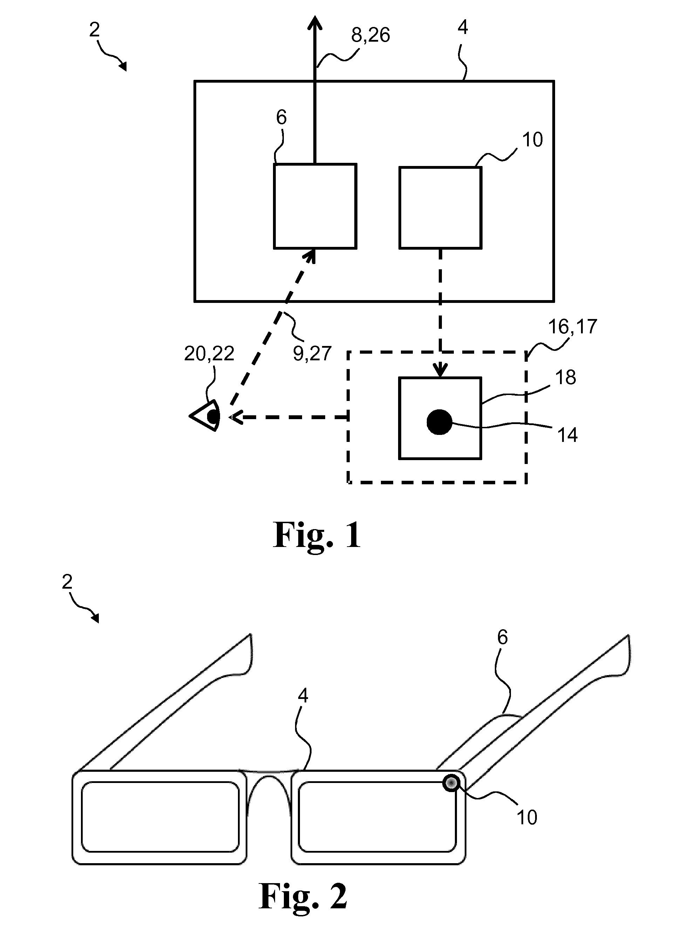

Fig. 1 schematically illustrates an exemplary head mountable device 2 for measuring eye movement of a user. The head mountable device 2 comprises a frame 4, a camera system 6, and a projection system 10. The camera system 6 and the projection system 10 are mounted on the frame 4.

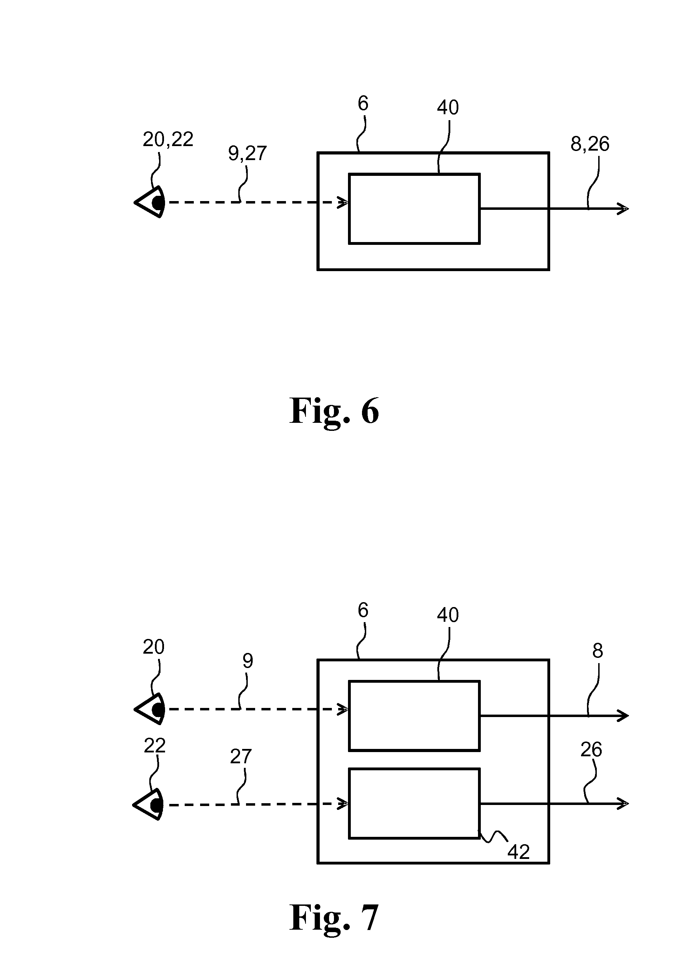

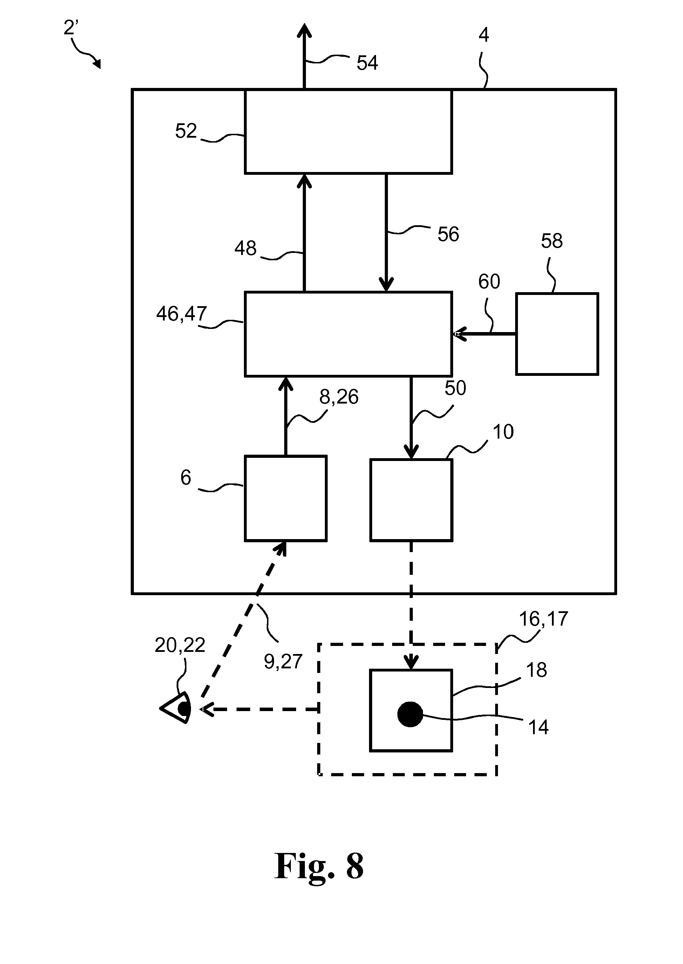

The camera system 6 comprises a first camera (Figs 7 and 8). The camera system 6 is configured to obtain a first set of images 8 of a first eye 20 of a user. Alternatively or additionally, the camera system 6 may be configured to obtain a second set of images 26 of a second eye 22 of the user. The camera system 6 detects images 9 of the first eye 20 and converts the images 9 of the first eye 20 to the first set of images 8 of the first eye 20. Alternatively and/or additionally, the camera system 6 detects images 27 of the second eye 22 and converts the images 27 of the second eye 22 to the second set of images 26 of the second eye 22.

The projection system 10 is configured for projecting a visible object 14 in a field of view of the user 16, 17, such as in a field of view 16 of the first eye 20 and/or in a field of view 17 of the second eye 22, when the user wears the head mountable device 2. The projection system 10 is configured to project a first projection 18 comprising the visible object 14. Furthermore, the projection system 10 is configured to perform a movement of the visible object 14 relative to the head mountable device 2.

Fig. 2 illustrates an exemplary head mountable device 2 for measuring eye movement of a user. The head mountable device 2 comprises a frame 4, a camera system 6, and a projection system 10. The camera system 6 and the projection system 10 is mounted on the frame 4.

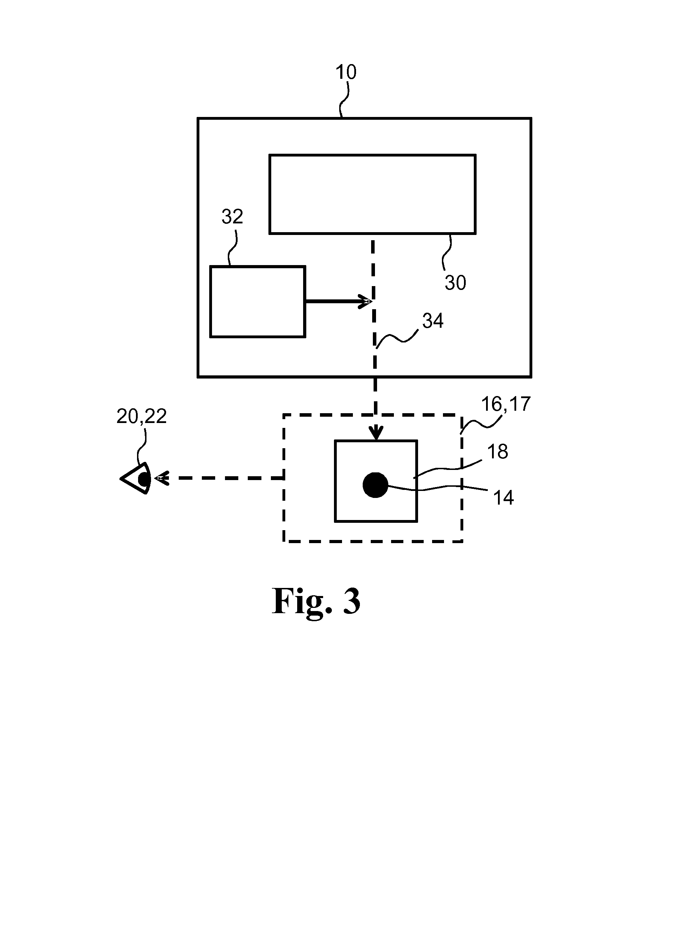

Fig. 3 schematically illustrates an exemplary projection system 10 for a head mountable device 2. The projection system 10 comprises a first projector 30 configured to project a first projection 18 comprising a visible object 14 in a field of view of the user 16, 17, such as in a field of view 16 of the first eye 20, and/or in a field of view 17 of the second eye 22, when the user wears the head mountable device 2. The projection system 10 is configured to perform a movement of the visible object 14 relative to the head mountable device. Fig. 3 depicts the projection system 10 comprising a first motor 32 configured to perform the movement of the visible object 14 by changing a first projection direction 34 of the first projector 30. For example, the first motor 32 may change the first projection direction 34 by changing the orientation of the first projector 30, and/or the first motor 32 may change the first projection direction 34 by changing orientation of a light guide, such as a mirror, a lens, and/or one or more optical fibre(s).

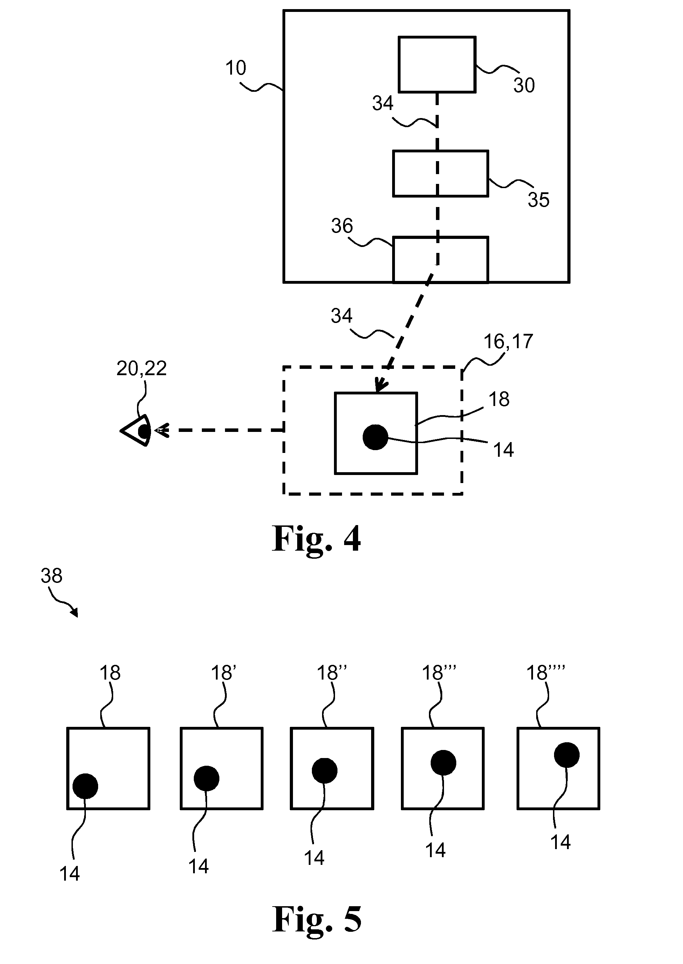

Fig. 4 schematically illustrates an exemplary projection system 10 for a head mountable device 2. The projection system 10 comprises a first projector 30 configured to project a first projection 18 comprising a visible object 14 in a field of view of the user 16, 17, such as in a field of view 16 of the first eye 20, and/or in a field of view 17 of the second eye 22, when the user wears the head mountable device 2. The projection system 10 is configured to perform a movement of the visible object 14 relative to the head mountable device 2. Fig. 4 depicts the projection system 10 comprising a projection mirror 36 configured to reflect the first projection 18. The projection mirror 36 may change the first projection direction 34 by reflecting the first projection 18. Furthermore, Fig. 4 depicts the projection system 10 comprising an optical lens 35 configured for changing a shape of the visible object 14. For example, the optical lens 35 may increase and/or decrease the size of the visible object 14.

In another exemplary projection system (not shown), the first motor 32 as described in relation to Fig. 3 may be implemented to change the orientation of the projection mirror 36 as described in relation to Fig. 4. Thus, the movement of the visible object 14 relative to the head mountable device 2 is achieved by movement of the projection mirror 36 thus changing the first projection direction 34, and the movement of the projection mirror 36 is facilitated by the first motor 36.

The first projector 30 may, as described in relation Fig. 3, perform the movement of the visible object 14 by changing the first projection direction 34, e.g. by providing a first motor 32. Alternatively and/or additionally, the movement of the visible object 14 may be performed by providing a sequence of projections 38. Fig. 5 schematically illustrates an exemplary sequence of projections 38. The first projector 30 may be configured to perform the movement of the visible object 14 by providing a sequence of projections 38. The sequence of projections 38 comprises a plurality of projections 18, 18', 18", 18"', 18"", including the first projection 18. The visible object 14 occupies different positions in one or more of the plurality of projections 18, 18', 18", 18"', 18"". Thereby, the movement of the visible object 14 is achieved by playing the sequence of projections 38. The plurality of projections 18, 18', 18", 18"', 18"" may be projections of images comprising the visible object 14. In Fig. 5 the sequence of projections 38 is shown as comprising a first projection 18, a second projection 18', a third projection 18", a fourth projection 18"', and a fifth projection 18"". It will be understood that the sequence of projections 38 may comprise any number of a plurality of projections.

Fig. 6 schematically illustrates an exemplary camera system 6 for a head mountable device 2. The camera system 6 comprises a first camera 40. The first camera 40 detects images 9 of a first eye 20 of a user and converts the images 9 of the first eye 20 to a first set of images 8 of the first eye 20. The first camera 40 converts the images 9 of the first eye 20 to the first set of images 8 of the first eye 20 with a first frame rate and a first resolution. Alternatively and/or additionally, the first camera 40 detects images 27 of a second eye 22 of the user and converts the images 27 of the second eye 22 to a second set of images 26 of the second eye 22. The first camera 40 converts the images 27 of the second eye 22 to a second set of images 26 of the second eye 22 with a second frame rate and a second resolution.

Fig. 7 schematically illustrates an exemplary camera system 6 for a head mountable device 2. The camera system 6 of Fig. 7 comprises a first camera 40 and a second camera 42. The first camera 40 detects images 9 of a first eye 20 and converts the images 9 of the first eye 20 to a first set of images 8 of the first eye 20. The first camera converts the images 9 of the first eye 20 to a first set of images 8 of the first eye 20 with a first frame rate and a first resolution. The second camera 42 detects images 27 of a second eye 22 and converts the images 27 of the second eye 22 to a second set of images 26 of the second eye 22. The second camera 42 converts the images 27 of the second eye 22 to a second set of images 26 of the second eye 22 with a second frame rate and a second resolution.

In relation to any of Figs. 6 and 7, the first camera 40 and/or the second camera 42 may be adapted to enable detection of eye saccades of the first eye 20 and/or second eye 30. For example, the first frame rate and/or the second frame rate may be higher than 125 fps. The first camera 40 and/or the second camera 42 may be able to detect electromagnetic radiation such as infrared radiation (IR), laser light, and/or coloured visible light, e.g. red, blue, green, and/or orange visible light. The first camera 40 and/or the second camera 42 may be able to detect electromagnetic radiation of a first light source (not shown).

Fig. 8 schematically illustrates an exemplary head mountable device 2' for measuring eye movement. The head mountable device 2' of Fig. 8 comprises a frame 4, a camera system 6 and a projection system 10 as the head mountable device 2 described in relation to Figs. 1 and 2. Furthermore the head mountable device 2' comprises a number of additional features, which individually and/or in combination may be added to the head mountable device 2 described in relation to Figs. 1 and 2. The head mountable device 2' further comprises a first processing unit 46 and/or a second processing unit 47, an interface 52, and a motion sensor 58. The camera system 6, the projection system 10, the first processing unit 46 and/or the second processing unit 47, the interface 52, and the motion sensor 58 are mounted on the frame 4.

The first processing unit 46 is configured to process the first set of images 8 and/or the second set of images 26, and provide a processing unit output 48 based on the first set of images 8 and/or the second set of images 26.

The second processing unit 47 is configured to control the projection system 10 to perform the movement of the visible object 14 relative to the head mountable device 2. The second processing unit 47 provides the projection system 10 with a projection system control signal 50, thus controlling the movement of the visible object 14. Alternatively and/or additionally, the projection system control signal 50 may provide the projection system 10 with instructions relating to projection such as the first projection 18. For example, the second processing unit 47 may provide the projection system 10 with images to be projected.

The first processing unit 46 and the second processing unit 47 may be separate processing units. However, the first processing unit 46 and the second processing unit 47 may be the same processing unit, e.g. the first processing unit 46.

The interface 52 provides a device output 54. The device output 54 may be based on the first set of images 8 and/or the second set of images 26. In the depicted example, the device output 54 is based on the processing unit output 48 which, as described, is based on the first set of images 8 and/or the second set of images 26. Furthermore, in the depicted example, the interface 34 provides a processing unit control signal 56. However, in other exemplary head mountable devices, the provision of a processing unit control signal 56 may be omitted. The processing unit control signal 56 may allow user control of the first processing unit 46 and/or the second processing unit 47 and/or the head mountable device 2' via an input device, such as a user interface, of the interface 52.

The motion sensor 58 is configured to detect movement of the head mountable device 2'. The first processing unit 46 is connected to the motion sensor 58. The motion sensor 58 provides a sensor output 60. The first processing unit 46 is configured to process the sensor output 60 from the first motion sensor 58, and the processing unit output 48 may be based on the sensor output 60. The motion sensor 58 may comprise one or more gyroscope(s) and/or one or more accelerometer(s).

The processing unit output 48 and/or the device output 54 may be indicative of one or more parameters of the user, such as an ophthalmologic parameter, a vestibular parameter, and/or a neurologic parameter.

The first processing unit 46 may compress and/or reduce the amount of data in the processing unit output 48. For example, in order for the interface 52 to transmit the device output 54, or a part of the device output 54, wirelessly, without substantial delay e.g. a delay of the order of 10 ms, the processing unit output 48 may be compressed and/or reduced. For example, the processing unit output 48 may comprise a first secondary set of images with a first secondary frame rate and a first secondary resolution, wherein the first secondary frame rate is smaller than the first frame rate and/or the first secondary resolution is smaller than the first resolution. Alternatively and/or additionally the processing unit output 30 may comprise a second secondary set of images with a second secondary frame rate and a second secondary resolution, wherein the second secondary frame rate is smaller than the second frame rate and/or the second secondary resolution is smaller than the second resolution.

Additionally and/or alternatively, the first processing unit 46 may be configured to compress an initial processing unit output based on the first set of images 8 and/or the second set of images 26, wherein the size of the processing unit output 46 is below 20%, such as 10%, such as 5% of the size of the initial processing unit output.

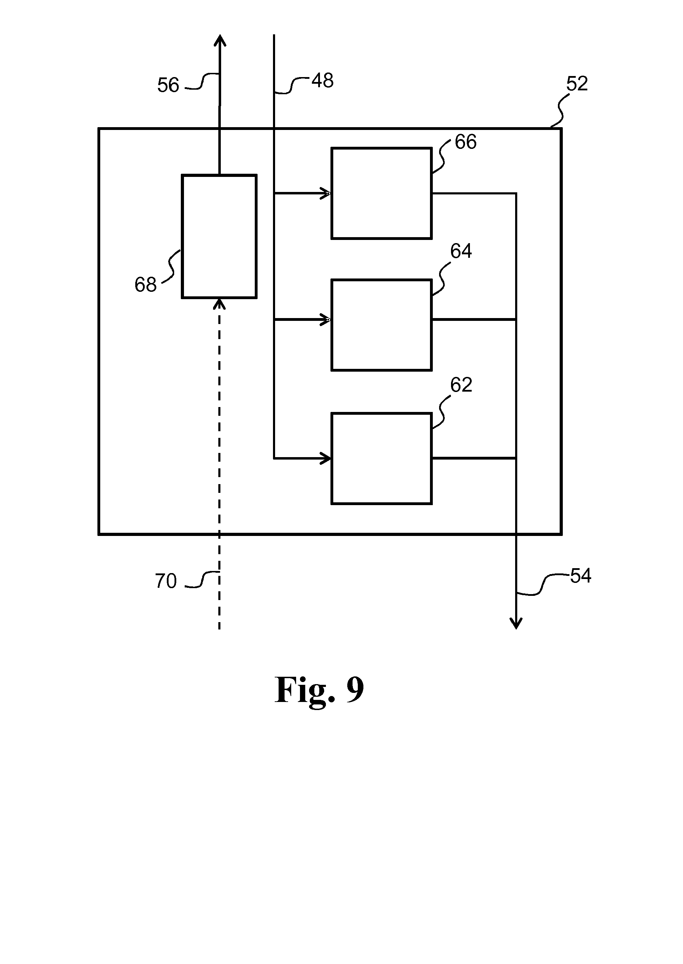

Fig. 9 schematically illustrates an exemplary interface 52. The interface 52 comprises a wireless transmitter unit 62, a first display 64, a speaker 66, and an input device 68. The interface 52 may in alternative configurations (not shown) comprise one or more of the wireless transmitter unit 62, the first display 64, the speaker 66 and the input device 68.

The wireless transmitter unit 62 receives the processing unit output 48, or part of the processing unit output 48, and transmits the device output 54, or a part of the device output 54, wirelessly to a wireless receiver (not shown). The wireless transmitter unit 62 may be a Bluetooth transmitter, a WiFi transmitter, a 3G transmitter and/or a 4G transmitter. The wireless transmitter unit 62 may further be configured to transmit the device output 54, or a part of the device output 54, with a low latency to enable live preview of the device output 54 in an external display. The latency may be less than 40 ms such as less than 20 ms such as less than 10 ms.

The first display 64 receives the processing unit output 48, or part of the processing unit output 48, and visually presents the device output 54, or a part of the device output 54, to a user or an operator of the device. The first display 64 may be an organic light emitting diode (OLED), an OLED display, a light emitting diode (LED), an LED display, and/or an e-ink display.

The speaker 66 receives the processing unit output 48, or part of the processing unit output 48, and audiologically presents the device output 54, or a part of the device output 54, to a user or an operator of the device.

The input device 68 enables control of the head mountable device 2, 2'. User interaction 70 is detected by the input device 68, and the input device 68 provides a control signal 56 to the first processing unit 46 and/or the second processing unit 47. The input device 68 may comprise a push button, a switch, and/or a touch display.

The device output 54 may be indicative of a positive/negative result of a test. For example, the device output 54 may comprise lighting up the first display 64 in a red colour if the test result is negative, and/or lighting up the first display 64 in a green colour if the test result is positive. For example, the device output 54 is indicative of an ophthalmologic parameter of the user, the device output 54 is indicative of a vestibular parameter of the user, and/or the device output 54 is indicative of a neurologic parameter of the user.

The device output 54 may comprise a plurality of output images based on the first set of images 8 and/or based on the second set of images 26. For example, the device output 54 may provide a live preview of the images 9, 27 of the first eye 20 and/or the second eye 22. The live preview may be transmitted wirelessly via the wireless transmitter 62 to an external display, e.g. a display of an external device, such as a tablet computer, a smart phone, or a laptop.

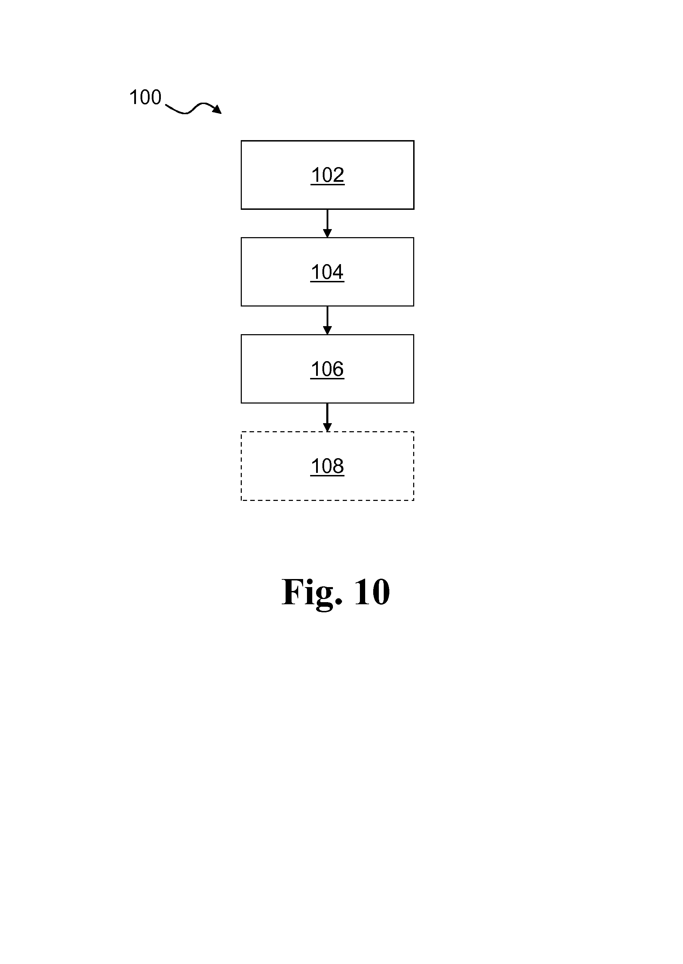

Fig. 10 shows a flow diagram of a method 100 for measuring eye movement. The method 100 may comprise using a head mountable device 2, 2', such as a head mountable device 2, 2' as described in relation to any of the previous figures. The method comprises obtaining 102 a first set of images of the first eye and/or a second set of images of the second eye; projecting 104 a first projection comprising a visible object in a field of view of the user, such as a field of view of the first eye and/or a field of view of the second eye; and performing 106 a movement of the visible object relative to the head mountable device.

Obtaining 102 the first set of images and/or the second set of images may be achieved by a camera system of the head mountable device, such as the camera system 6 described in relation to previous figures. The first set of images and/or the second set on images may be obtained with a respective first frame rate and/or second frame rate enabling detection of eye saccades of the respective first eye and/or second eye, e.g. a first frame rate and/or a second frame rate higher than 125 fps.

Projecting 104 the first projection may be achieved by a projection system of the head mountable device, such as the projection system 10 described in relation to the previous figures. The first projection may be a part of a sequence of projections.

Performing 106 the movement of the visible object may comprise changing an orientation of a first projector projecting the first projection and/or performing 106 the movement of the visible object may comprise changing a first projection direction of the first projection and/or performing 106 the movement of the visible object may comprise projecting a sequence of projections wherein the visible object occupy different positions in one or more of the projections of the sequence of projections.

Additionally, the method 100 may comprise providing 108 a device output based on the first set of images and/or the second set of images.

The device output provided 108 may be indicative of one or more parameters of the user, e.g. a vestibular parameter of the user, an ophthalmologic parameter of the user, and/or a neurologic parameter of the user. The device output may further be indicative of a test result, such as a vestibular test, an ophthalmologic test and/or a neurologic test. The device output may be provided 108 via an audiologic output, a visual output, and/or wireless transmission to an external device.

The method 100 may furthermore comprise mounting (not shown) the head mountable device to a head of the user, and/or detecting (not shown) movement of the head mountable device.

Mounting of the head mountable device to a head of the user may be performed by an operator, and may involve fastening the head mountable device to the head of the user to avoid movement of the head mountable device relative to the head of the user. If the device is tightly fixed to the head, moving the head of the user involves movement of the head mountable device. Thus, the movement of the device corresponds to the movement of the head of the user. Detecting of the movement of the head mountable device is therefore indicative of the moving of the head of the user.

Embodiments and aspects are disclosed in the following items:
Item 1. A head mountable device for measuring eye movement of a user, the head mountable device comprising:
   - a frame;
   - a camera system comprising a first camera, wherein the camera system is configured to obtain a first set of images of a first eye of the user;
   - a projection system for projecting a visible object in a field of view of the first eye when the user wears the head mountable device, and where the projection system is configured to perform a movement of the visible object relative to the head mountable device, the projection system comprising a first projector configured to project a first projection comprising the visible object.
Item 2. Head mountable device according to item 1, wherein the projection system comprises a first motor configured to perform the movement of the visible object by changing a first projection direction of the first projector.
Item 3. Head mountable device according to any of items 1-2, wherein the projection system comprises a projection mirror configured to reflect the first projection.
Item 4. Head mountable device according to any of the preceding items, wherein the first projector is configured to perform the movement of the visible object by providing a sequence of projections including the first projection.
Item 5. Head mountable device according to any of the preceding items, wherein the first projection is an image.
Item 6. Head mountable device according to any of items 1-4, wherein the first projection is a dot having a diameter of more than 0.5 cm.
Item 7. Head mountable device according to any of the preceding items, wherein the camera system is configured to obtain a second set of images of a second eye of the user.
Item 8. Head mountable device according to item 7, wherein the camera system comprises a second camera configured to obtain the second set of images.
Item 9. Head mountable device according to any of the preceding items, wherein the projection system is further configured for projecting a visible object in a field of view of the second eye when the user wears the head mountable device.
Item 10. Head mountable device according to any of the preceding items, wherein the first set of images is configured to be obtained with a first frame rate and, wherein the first frame rate is selected such as to enable detection of eye saccades of the first eye.
Item 11. Head mountable device according to any of the preceding items, wherein the head mountable device comprises a first processing unit configured to process the first set of images and providing a processing unit output based on the first set of images.
Item 12. Head mountable device according to any of the preceding items, wherein the head mountable device comprises a second processing unit configured to control the projection system to perform the movement of the visible object relative to the head mountable device.
Item 13. Head mountable device according to any of the preceding items, wherein the head mountable device comprises an interface for providing a device output based on the first set of images.
Item 14. Head mountable device according to item 13, wherein the interface comprises one or more of a display, a speaker, and a wireless transmitter unit.
Item 15. Head mountable device according to any of the preceding items, wherein the head mountable device comprises a first motion sensor configured to detect movement of the head mountable device.
Item 16. Head mountable device according to any of the preceding items, wherein the first projector is a laser pointer and/or a video projector.
Item 17. Head mountable device according to any of the preceding items, wherein the projection system comprises an optical lens configured for changing a shape of the visible object.
Item 18. A method for measuring eye movement of a user wearing a head mountable device comprising a frame; a camera system comprising a first camera, and a projection system comprising a first projector, the method comprising:
   - obtaining a first set of images of a first eye of the user by the camera system;
   - projecting a first projection comprising a visible object in a field of view of the first eye by the projection system; and
   - performing a movement of the visible object relative to the head mountable device by the projection system.
Item 19. A head mountable device for measuring eye movement of a user, the head mountable device comprising:
   a frame;
   a camera system comprising a first camera, wherein the camera system is configured to obtain a first set of images of a first eye of the user; and
   a projection system for projecting a first projection comprising a visible object in a field of view of the first eye when the user wears the head mountable device, wherein the projection system is configured to move the visible object relative to the head mountable device.
Item 20. The head mountable device according to item 19, wherein the projection system comprises a first projector, and a first motor configured to move the visible object by changing a first projection direction of the first projector.
Item 21. The head mountable device according to item 19, wherein the projection system comprises a projection mirror configured to reflect the first projection.
Item 22. The head mountable device according to item 19, wherein the projection system is configured to move the visible object by providing a sequence of projections including the first projection.
Item 23. The head mountable device according to item 19, wherein the first projection comprises an image.
Item 24. The head mountable device according to item 19, wherein the first projection is a dot having a diameter of more than 0.5 cm.
Item 25. The head mountable device according to item 19, wherein the camera system is configured to obtain a second set of images of a second eye of the user.
Item 26. The head mountable device according to item 25, wherein the camera system comprises a second camera configured to obtain the second set of images.
Item 27. The head mountable device according to item 19, wherein the projection system is further configured for projecting a second projection in a field of view of the second eye when the user wears the head mountable device.
Item 28. The head mountable device according to item 19, wherein the first camera is configured to obtain the first set of images with a first frame rate, and wherein the first frame rate is selected such as to enable detection of eye saccades of the first eye.
Item 29. The head mountable device according to item 19, further comprising a first processing unit configured to process the first set of images, and to provide a processing unit output based on the first set of images.
Item 30. The head mountable device according to item 19, further comprising a second processing unit configured to control the projection system to move the visible object relative to the head mountable device.
Item 31. The head mountable device according to item 19, further comprising an interface for providing a device output based on the first set of images.
Item 32. The head mountable device according to item 31, wherein the interface comprises one or more of a display, a speaker, and a wireless transmitter unit.
Item 33. The head mountable device according to item 19, wherein the head mountable device comprises a first motion sensor configured to detect a movement of the head mountable device.
Item 34. The head mountable device according to item 19, wherein the projection system comprises a laser pointer and/or a video projector.
Item 35. The head mountable device according to item 19, wherein the projection system comprises an optical lens configured for changing a shape of the visible object.
Item 36. A method for measuring eye movement of a user wearing a head mountable device comprising a frame, a camera system comprising a first camera, and a projection system comprising a first projector, the method comprising:
   obtaining a first set of images of a first eye of the user by the camera system;
   projecting a first projection comprising a visible object in a field of view of the first eye by the projection system; and
   moving the visible object relative to the head mountable device by the projection system.

Although particular features have been shown and described, it will be understood that they are not intended to limit the claimed invention, and it will be made obvious to those skilled in the art that various changes and modifications may be made without departing from the spirit and scope of the claimed invention. The specification and drawings are, accordingly to be regarded in an illustrative rather than restrictive sense.

The claimed invention is intended to cover all alternatives, modifications and equivalents.

### LIST OF REFERENCES

- 2, 2': head mountable device
- 4: frame
- 6: camera system
- 8: first set of images
- 9: image(s) of first eye
- 10: projection system
- 14: visible object
- 16: field of view of first eye
- 17: field of view of second eye
- 18: first projection
- 18': second projection
- 18": third projection
- 18"': fourth projection
- 18"": fifth projection
- 20: first eye
- 22: second eye
- 26: second set of images
- 27: image(s) of second eye
- 30: first projector
- 32: first motor
- 34: first projection direction
- 35: optical lens
- 36: projection mirror
- 38: sequence of projections
- 40: first camera
- 42: second camera
- 46: first processing unit
- 47: second processing unit
- 48: processing unit output
- 50: projection system control signal
- 52: interface
- 54: device output
- 56: processing unit control signal
- 58: motion sensor
- 60: motion sensor signal
- 62: wireless transmitter unit
- 64: first display
- 66: speaker
- 68: input device
- 70: user interaction
- 100: method
- 102: obtaining first set of images
- 104: projecting first projection
- 106: performing movement of visible object
- 108: providing device output

## Claims

1. A head mountable device for measuring eye movement of a user, the head mountable device comprising:
- a frame;
- a camera system comprising a first camera, wherein the camera system is configured to obtain a first set of images of a first eye of the user;
- a projection system for projecting a visible object in a field of view of the first eye when the user wears the head mountable device, and where the projection system is configured to perform a movement of the visible object relative to the head mountable device, the projection system comprising a first projector configured to project a first projection comprising the visible object.

2. Head mountable device according to claim 1, wherein the projection system comprises a first motor configured to perform the movement of the visible object by changing a first projection direction of the first projector.

3. Head mountable device according to any of claims 1-2, wherein the projection system comprises a projection mirror configured to reflect the first projection.

4. Head mountable device according to any of the preceding claims, wherein the first projector is configured to perform the movement of the visible object by providing a sequence of projections including the first projection.

5. Head mountable device according to any of the preceding claims, wherein the first projection is an image.

6. Head mountable device according to any of claims 1-4, wherein the first projection is a dot having a diameter of more than 0.5 cm.

7. Head mountable device according to any of the preceding claims, wherein the camera system is configured to obtain a second set of images of a second eye of the user.

8. Head mountable device according to any of the preceding claims, wherein the projection system is further configured for projecting a visible object in a field of view of the second eye when the user wears the head mountable device.

9. Head mountable device according to any of the preceding claims, wherein the head mountable device comprises a first processing unit configured to process the first set of images and providing a processing unit output based on the first set of images.

10. Head mountable device according to any of the preceding claims, wherein the head mountable device comprises a second processing unit configured to control the projection system to perform the movement of the visible object relative to the head mountable device.

11. Head mountable device according to any of the preceding claims, wherein the head mountable device comprises an interface for providing a device output based on the first set of images.

12. Head mountable device according to any of the preceding claims, wherein the head mountable device comprises a first motion sensor configured to detect movement of the head mountable device.

13. Head mountable device according to any of the preceding claims, wherein the first projector is a laser pointer and/or a video projector.

14. Head mountable device according to any of the preceding claims, wherein the projection system comprises an optical lens configured for changing a shape of the visible object.

15. A method for measuring eye movement of a user wearing a head mountable device comprising a frame; a camera system comprising a first camera, and a projection system comprising a first projector, the method comprising:
- obtaining a first set of images of a first eye of the user by the camera system;
- projecting a first projection comprising a visible object in a field of view of the first eye by the projection system; and
- performing a movement of the visible object relative to the head mountable device by the projection system.
